# EUROPEAN PATENT SPECIFICATION

(11) **EP 4 237 216 B1**
(45) Date of publication and mention of the grant of the patent: **27.08.2025**
(21) Application number: 21806824.5
(22) Date of filing: 28.10.2021
(51) Int. Cl.: B29C 33/42, A61B 17/3207, A61B 17/00, A61B 17/12, A61B 17/22, A61M 25/10, B29C 67/00, A61B 18/14, A61B 17/32, B29C 49/00, B29L 31/00

(54) **MOLD FOR FORMING AN EXPANDABLE BALLOON FOR INTRALUMINAL USE AND METHOD**
FORM ZUR HERSTELLUNG EINES EXPANDIERBAREN BALLONS ZUR INTRALUMINALEN VERWENDUNG UND VERFAHREN
MOULE POUR FORMER UN BALLONNET EXPANSIBLE POUR UNE UTILISATION INTRALUMINALE ET PROCÉDÉ

(30) Priority: 30.10.2020 IT 202000025915; 30.10.2020 IT 202000025918
(43) Date of publication of application: 06.09.2023
(73) Proprietor: I-VASC S.r.l., 20123 Milano (IT)
(72) Inventor: SALERNO, Mario, 20123 Milano (IT)
(74) Representative: Crippa, Paolo Ernesto
(86) International application number: PCT/IB2021/059976
(87) International publication number: WO 2022/090991

(56) References cited:
- EP-A1- 0 597 506
- EP-A2- 0 937 482
- WO-A1-2010/122991
- US-A1- 2010 102 490
- US-A1- 2012 253 278
- US-A1- 2015 091 221
- US-A1- 2020 276 417

## Description

### . Field of the invention

**.** The present invention relates to the technical domain of expandable balloons for intraluminal use.

**.** The object of the present invention is a mold for forming such an expandable balloon.

**.** The present invention further relates to a manufacturing method for an expandable balloon and an expandable balloon having a concave portion.

**.** Furthermore, the present invention relates to a method for collecting material from a lumen of a patient's body.

### . Background art

**.** Various types of expandable balloons for medical-surgical applications are generally known. These balloons are typically intended to be inserted in a collapsed configuration inside a lumen of a patient's body and then expanded radially inside the lumen.

**.** Document US-6176698 shows a known mold solution with tapered walls for forming an expandable balloon. Further examples of molds for making expandable balloons are known from documents US-4963313, US-5334146, EP-0937482A2 and US-2020/276417.

**.** The known solutions mentioned above are all aimed at making an expandable balloon having a substantially cylindrical convex portion tapering towards the ends.

**.** For example, patent EP-2120738-B1 of the same Applicant discloses an embodiment of a catheter provided with expandable balloons which expand to isolate from the blood circulation an annular chamber inside the blood vessel in which to inject the sclerosing drug in a controlled manner. The presence of such radially expandable balloons, which isolate an annular chamber between the catheter shaft and the vessel wall, avoids having to inject the drug into the entire section of the vessel stretch to be treated, allowing the drug to be injected only in one annular area of the blood vessel in contact with the wall. The device disclosed therein allows performing empty vein sclerotherapy, without blood, therefore without plasma proteins which can neutralize the pharmacological activity of the sclerosing drug; this allows a fine control of the concentration of the drug injected on the internal wall of the vein; it allows positioning at 360° the drug on the entire internal vein surface and it also allows managing a time of drug contact with the vein wall. The drug-to-part contact time is critical to allow the drug to penetrate the internal wall thickness of the vein and generate an inflammatory vein repair response.

**.** The need is therefore felt to provide a mold having features such as to allow the production of an expandable balloon suitable for use in the medical-surgical field of different shape with respect to known solutions.

**.** At the same time, the need is felt to provide an alternative solution of expandable balloons suitable for intraluminal use in the medical-surgical field with improved functionality with respect to known solutions.

### . Solution

**.** An object of the present invention is to obviate the drawbacks of the known art described up to now.

**.** This and other objects are achieved with a mold according to claim **1,** as well as with a method according to claim 7.

**.** Some advantageous embodiments are the subject of the dependent claims.

**.** It is clear that the appended claims are an integral part of the present description.

### . Drawings

**.** Further features and advantages of the invention will become apparent from the description provided below of preferred exemplary embodiments thereof, given by way of nonlimiting example, with reference to the accompanying drawings, in which:

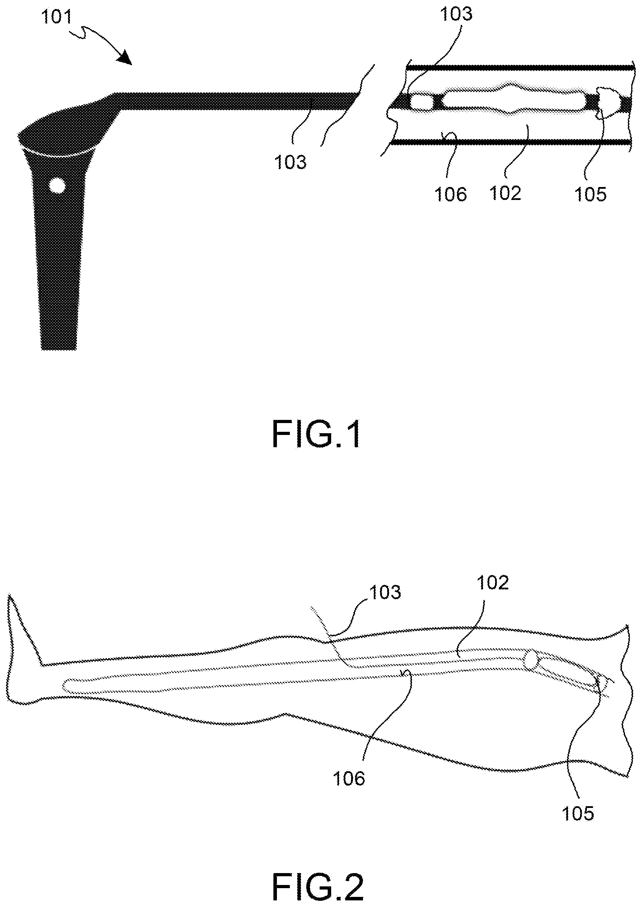

- figure 1 is a diagrammatic view of an expandable balloon according to an embodiment associated with a vascular catheter;
- figure 2 schematically shows an expandable balloon according to an embodiment associated with a vascular catheter;

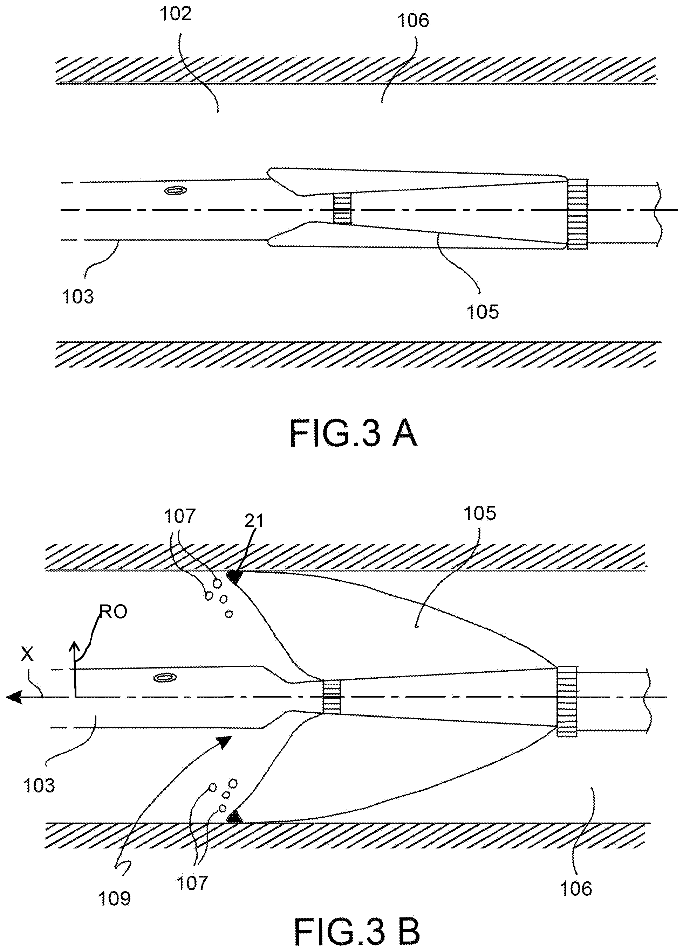

- figure 3 A shows an expandable balloon when in a collapsed configuration, according to an embodiment;
- figure 3 B shows the expandable balloon of figure 3 A when in an expanded configuration;
- figure 4 A shows an expandable balloon when in a collapsed configuration, according to an embodiment;
- figure 4 B shows the expandable balloon of figure 4 A when in an expanded configuration;
- figure 5 shows a possible step of a method, according to an operating mode;
- figures 6 A to 6 F show some steps of a method, according to an operating mode, which uses a die as well as a mold according to an embodiment;
- figures 7 A and 7 B show some steps of a method, according to an operating mode, which uses a die as well as a mold according to an embodiment;
- figures 8 A to 8 D show some steps of a method, according to an operating mode, which uses a die as well as a mold according to an embodiment;
- Figures 9 A to 9 D show some method steps, according to an operating mode

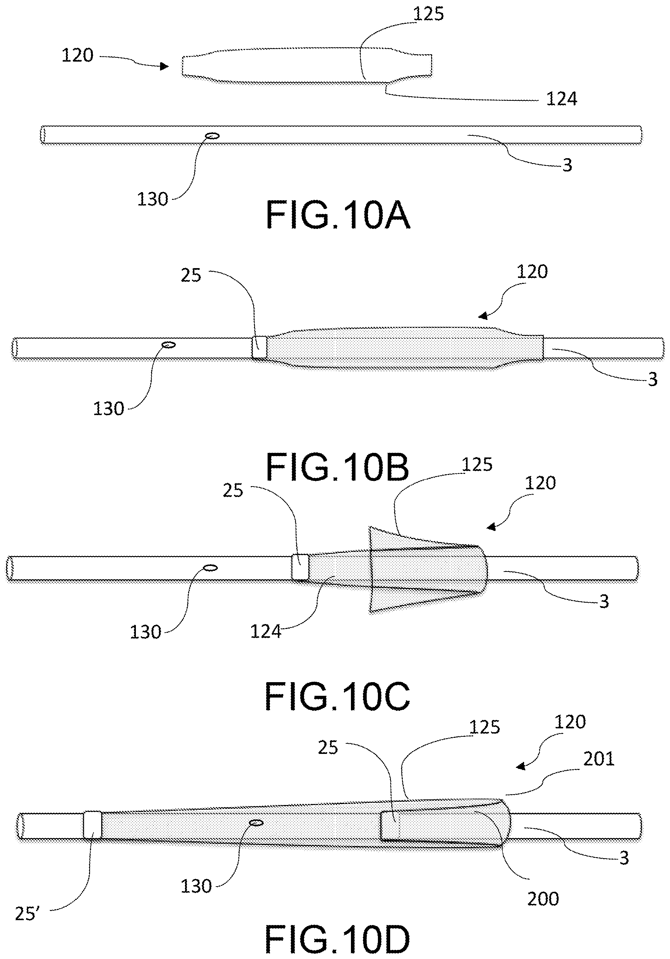

- figures 10A to 10D show some method steps, according to an operating mode, in which the parison, having an external surface, and an opposite internal surface delimiting an internal parison cavity, is fitted to an intraluminal element, such as a shaft of a catheter, in which a first parison end is tightly fastened on the intraluminal element leaving the second end free, so as to overturn the internal surface of the parison and fastening the second sealing end on the intraluminal element from an opposite part with respect to the first free end so that the parison cavity is delimited by the external parison surface and the intraluminal element;

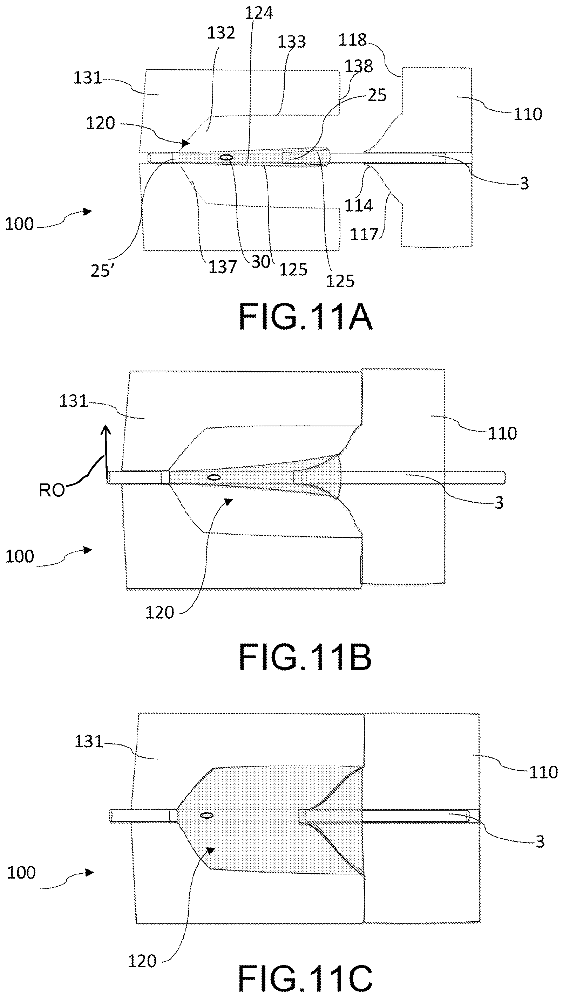

- figures 11A to 11C show some method steps, according to an operating mode, in which the parison mounted on the intraluminal element is inserted in the mold, the parison is expanded, and thermoformed;

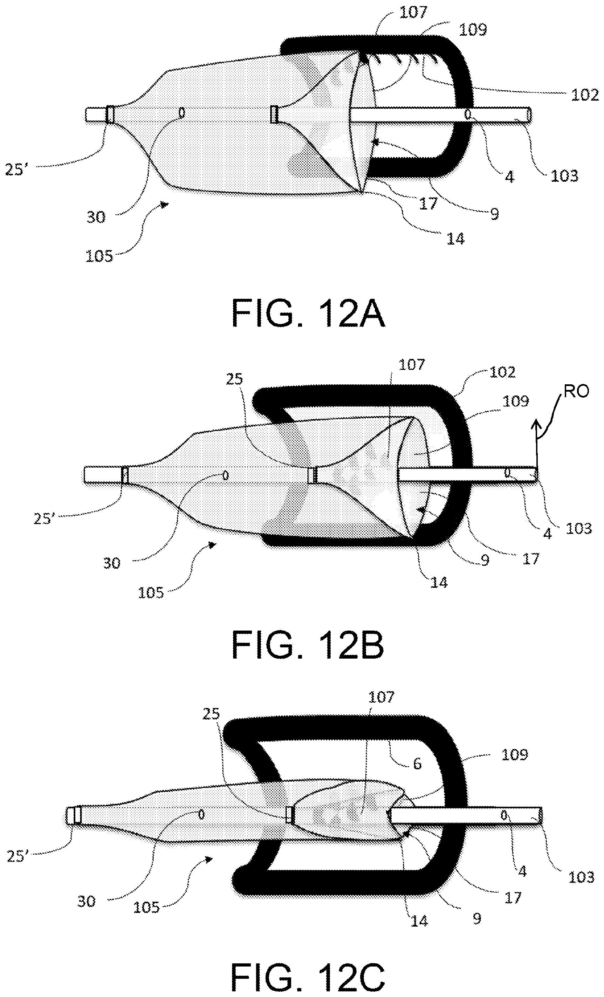

- figures 12 A to 12C diagrammatically show the expandable balloon according to the present invention, mounted on the intraluminal element and arranged inside a partially sectioned blood vessel, and is in an expanded configuration in figure 12 A, in which the expandable balloon is visible advancing to remove material from the internal wall of the stretch of the vessel with the contact portion and collect the material in the concave portion; in figure 12 B, the abrasion element is in the rest configuration with the contact portion spaced from the internal wall and with the material removed in the collection portion; and in figure 12 C, the abrasion element is in the rest configuration with the contact portion near the intraluminal element so as to retain the collected material in the collection portion, preventing tissue and pharmacological agent residues from being circulated in the bloodstream.

### . Detailed description of some embodiments

**.** In accordance with a general embodiment, a mold 100 is included for forming an expandable balloon 105. The expandable balloon 105 is suitable for medical-surgical applications. For example, the expandable balloon 105 is adapted to be mounted on an intraluminal element 103, for example a catheter shaft 103 of a vascular catheter 101. In accordance with a preferred embodiment, the expandable balloon 105 comprises a concave portion of the balloon 109 for collecting material 107, for example debris 107, from the lumen 102 of a blood vessel.

**.** The mold 100 comprises a die 110. The die 110 comprises a first wall 111 at least partially delimiting a concave portion 112 of a mold cavity 113. The inclusion of said concave portion 112 of the mold cavity 113 allows to form said concave balloon portion 109 of the expandable balloon 105.

. The first wall 111 of the die 110 comprises a protrusion 114 adapted to project cantilevered in said mold cavity 113 forming a free protrusion end 115 and a protrusion root 116 opposite said free end. According to the invention, said protrusion 114 is made in the form of a small cylinder.

**.** The first wall 111 of the die 110 further comprises a mold tapered surface 117 tapering as it approaches said protrusion root 116 of the protrusion 114. In other words, the extension of the tapered surface 107 of the first wall 111 of the die 110 is gradually and progressively reduced as it approaches the protrusion 114.

**.** By virtue of the inclusion of such a die 110 having such a first wall 111 it is possible to at least partially delimit a mold cavity 113 having a concave portion 112, so as to allow the formation of an expandable balloon 105 provided with a concave balloon portion 109.

**.** The forming of the expandable balloon 105 thus made determines that at least when in an expanded configuration the expandable balloon 105 comprises a concave balloon portion 109.

**.** The concave balloon portion 109 can be indistinguishable when the expandable balloon 105 is in a collapsed configuration to manifest when the expandable balloon 105 is in an expanded configuration.

**.** The expandable balloon 105 preferably has a thin wall and can be obtained starting from an internally hollow preform 120 or parison 120. The cavity 121 of the parison 120 is preferably adapted to receive said protrusion 114 of the first wall 111 of the die 110.

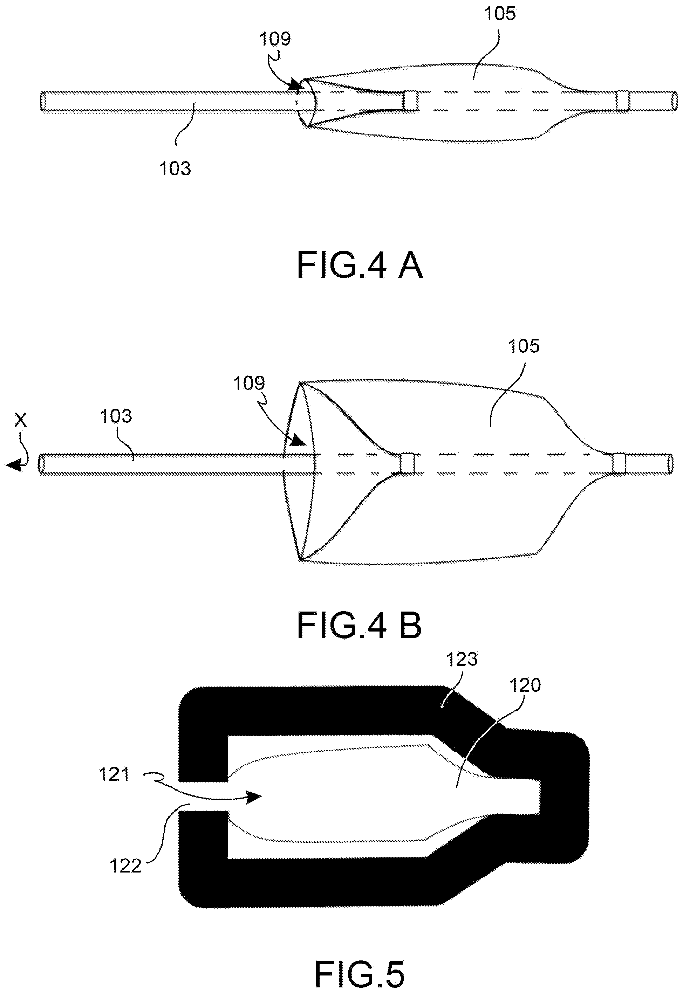

**.** As shown for example in **figure 5****,** the parison 120 can be made by injection molding inside a convex pre-mold 123. The parison 120 can be made by extrusion.

**.** The mold 100 comprising the die 110 can be suitable for a blow-molding forming process. One or more blowing channels 122 can be provided for blowing inflation fluid in the cavity 121 of the parison 120 when the parison 120 is received inside the mold cavity 113.

**.** The mold 100 comprising the die 111 can be suitable for an injection molding process.

**.** In accordance with an embodiment, said protrusion 114 has a substantially cylindrical shape. The term "substantially cylindrical" is intended to indicate a substantially cylindrical shape even though the side walls can be tapered to allow the molded piece to be drafted from the die 110, and for example the substantially cylindrical side walls can form a convergence angle less than or equal to 3°, where the convergence is in the direction of the free end 115 of the protrusion 114.

**.** In accordance with an embodiment, the protrusion root 116 is joined to the tapered surface 117. For example, an annular edge can be included between the protrusion root 119, which is preferably substantially cylindrical, and the tapered surface 117, which is preferably substantially frustoconical, or an annular draft connection can be included. The angle formed between the tapered surface 117 and the protrusion 115 is an obtuse angle.

**.** The protrusion 115 and the tapered surface 117 are made in a single piece. In accordance with an embodiment, the first wall 111 of the die 110 is preferably made in a single piece.

**.** In accordance with a preferred embodiment, the tapered surface 117 of the die 110 is frustoconical.

**.** The inclusion of said tapered surface 117 of the die 110 allows to obtain a concave portion 112 of the internal cavity 113 having an acute annular chamber, particularly when included in combination with a counter-die 131 forming an acute angle with the tapered wall 117 of the die 111.

**.** In accordance with a general embodiment, a mold 100 is provided comprising a die 110 according to any one of the preceding claims.

**.** Said mold 100 further comprises at least one counter-die 131 at least partially delimiting a convex portion 132 of said mold cavity 113.

**.** The mold cavity 113 preferably has a cylindrical extension. In other words, the mold cavity 113 preferably has a cylindrical geometry around a definable axis which preferably passes through or coincides with the longitudinal extension direction of the protrusion 114 of the die 110. Preferably, the protrusion 114 of the die 110 is aligned with the longitudinal extension axis of the mold cavity 113.

**.** In accordance with a preferred embodiment, the counter-die 131 of the mold 100 comprises a second tapered surface 137 tapering in a direction concordant with that of the first tapered surface 117 of the first wall 111 of the die 110.

**.** In accordance with an embodiment, the second tapered surface 137 of the counter-die 131 is parallel to the first tapered surface 117 of the first wall 111 of the die 110.

**.** In accordance with an embodiment, the first tapered surface 117 and the second tapered surface 137 face each other and face the mold cavity 113, delimiting therebetween a substantially concave volume having a concavity facing the first tapered surface 117 of the die 110.

**.** In accordance with an embodiment, the counter-die 131 forms an acute angle with the tapered surface 117 of the first wall 111 of the die 110. Preferably, the counter-die 131 comprises at least one side wall 133, substantially cylindrical, connected to the second tapered surface 137 of the counter-die 131, in which said at least one side wall 133 of the counter-die 131, when the counter-die 131 abuts against the die 110, forms an acute angle with the first tapered surface 117 of the first wall 111 of the die 110.

**.** Preferably, the die 110 comprises an abutment portion 118 and the counter-die 131 comprises a counter-abutment portion 138, said abutment portion 118 and said counter-abutment portion 138 are adapted to mutually abut to delimit the mold cavity 113 of the mold 100.

**.** The mold 100 can be a closed mold. Preferably, burr channels are included for the formation of burrs resulting from the flow of material during the forming by molding.

**.** The mold 100 can be an open mold. Preferably, the expandable balloon 105 thus obtained will comprise at least one convex barrel-like surface as a result of the flow of material out of the mold cavity during the forming by molding.

**.** In accordance with an embodiment, the die 110 substantially forms the core for at least one concave balloon portion 109 of an expandable balloon 105.

**.** In accordance with an embodiment, said counter-die 131 comprises a first channel wall delimiting the inlet of a first blowing channel 122b. In accordance with an embodiment, said first channel wall is connected to said second tapered surface 137. In accordance with an embodiment, said die 110 comprises a second channel wall delimiting the inlet of a second channel 122a. In accordance with an embodiment, said second channel of channel 122a is made inside said protrusion 114. In accordance with an embodiment, the first channel 122a and the second channel 122b are configured to accommodate the intraluminal element 103 on which the parison 120 is mounted to form the expandable balloon 105.

**.** The mold 100 as well as the die 110 and the counter-die 131 can be provided with heating elements to heat the parison 120.

**.** In accordance with a general embodiment, an expandable balloon 105 suitable for medical-surgical applications comprising a concave portion 109 is provided. Preferably, the expandable balloon 105 is suitable for intraluminal applications, such as the sclerosing treatment of the varices of a blood vessel 102.

**.** Said expandable balloon 105 is made using a die 110 according to any one of the previously described embodiments.

**.** Preferably, said expandable balloon 105 is made using a mold 100 according to any one of the previously described embodiments.

**.** The inclusion of said concave portion 109 of the expandable balloon 105 allows performing a collection of material 107, for example debris 107, from a lumen 102 of a patient's body, such as a lumen 102 of a blood vessel. For example, material 107 removed from the internal wall of a blood vessel 102 can be collected from the concave portion 109 of the expandable balloon 105. The collection can occur by translating, in other words by advancing, the expandable balloon 105 inside the lumen 102 of a blood vessel, taking care to face the concave portion 109 towards the advancement direction of the expandable balloon 105 inside the lumen 102.

**.** In accordance with an embodiment, said expandable balloon 105 comprises at least one first surface 17 adapted to face said intraluminal element 103. The at least one first surface 17 is adapted to face a fluid inside the blood vessel. The at least one first surface 17 at least in said expanded configuration, forms said at least one concave portion 109.

**.** In accordance with an embodiment, said expandable balloon 105 comprises at least one contact portion 14 adapted to remove material 107 from an internal wall 6 of a stretch of the blood vessel lumen 102.

**.** In accordance with an embodiment, said expandable balloon 105 is configured to expand with respect to said intraluminal element 103 at least in a radial direction RO transverse to the longitudinal extension direction of the vessel between at least one rest configuration and at least said expanded configuration. Said radial direction RO being transverse to the longitudinal extension direction of the intraluminal element 103.

**.** In accordance with an embodiment, between said contact configuration and said rest configuration, said contact portion 14 approaches said intraluminal element 103 closing said concave portion 109 in the direction of said intraluminal element 103 so as to retain the removed material 107.

**.** In accordance with an embodiment, said expandable balloon 105 comprises an attachment portion circumferentially connectable to the intraluminal element 103, in which said first surface 17 extends between said attachment portion and said contact portion 14.

**.** In accordance with an embodiment, said first surface 17 is substantially frustoconical.

**.** The contact portion 14 is preferably a surface having a substantially circumferential extension. For example, the contact portion 14 can be formed by a circumferential edge. In accordance with an embodiment, the contact portion 14 comprises a circumferential edge of the expandable balloon 105.

**.** In accordance with an embodiment, said contact portion 14 can have an increased section 21 with respect to the rest of the expandable balloon 105 and the increased section forms a reinforcing element 21. In accordance with an embodiment, said reinforcing element 21 comprises a sharp edge. In accordance with an embodiment, said expandable balloon 105 comprises a sharp edge comprising said contact portion 14.

**.** In accordance with an embodiment, said contact portion 14 preferably has a surface treatment aimed at increasing the roughness thereof. For example, the contact portion 14 can be corrugated or pleated, to favor the abrasive power on the internal wall 6. For example, the contact portion 14 can comprise surface knurling processing.

**.** In accordance with a preferred embodiment, the expandable balloon 105 is fitted on the intraluminal element 103, and the intraluminal element 103 comprises at least one inflation opening 30 in fluid communication with the inside of the expandable balloon 105 to introduce in or extract from the expandable balloon 105 the inflation fluid 18 so as to reversibly expand or contract the expandable balloon 105 between the rest configuration and the contact configuration.

**.** In accordance with an embodiment, the expandable balloon 105 has a longitudinal balloon extension axis around which the balloon extends. In accordance with an embodiment, said balloon 105 extends with cylindrical symmetry around said balloon longitudinal extension axis. In accordance with an embodiment, said radial direction RO is transverse with respect to said balloon longitudinal extension axis.

**.** In accordance with an embodiment, the expandable balloon 105 is tightly fastened on said intraluminal element 103 with a first fastening element 25 and a second fastening element 25' so as to define a fluid-tight chamber in fluid connection with said inflation opening 30 between an internal surface of the expandable balloon 105 and a surface of the intraluminal element 103 between the first fastening element 25 and the second fastening element 25', in which said concave portion 109 extends from said first fastening element 25 to said contact portion 14. In accordance with an embodiment, said expandable balloon 105 has a first attachment portion and a second attachment portion configured to be connected to said intraluminal element 3 by means of said first fastening element 25 and said second fastening element 25', in which said first attachment portion and said second attachment portion are circumferential, preferably cylindrical portions adapted to accommodate the intraluminal element 103, in which said first attachment portion and said second attachment portion are spaced apart by an attachment distance D along a longitudinal extension direction of the intraluminal element 103 or along said balloon longitudinal extension axis around which the balloon extends. In accordance with an embodiment, said attachment distance D is between 20 mm and 150 mm.

**.** In accordance with an embodiment, in which said concave portion 109, at least in said contact configuration, has an at least partially conical shape. In accordance with an embodiment, the collection portion 9 is joined to the contact portion 14 so that the material 107 removed from the contact portion 14 is directed in the collection portion avoiding interfering with the contact portion 14. In accordance with an embodiment, at least in the contact configuration, the collection portion 9 forms a funnel-shaped portion configured to facilitate the collection of the removed material 7 advancing said expandable balloon 105 inside the blood vessel along an advancement direction X. In accordance with an embodiment, the collection portion 9 and the contact portion 14 are seamlessly integrated.

**.** In accordance with an embodiment, when said balloon is in said expanded configuration, a projection of the concave portion 109 on the longitudinal extension axis of the balloon defines a segment having a concave portion length C. In accordance with an embodiment, said concave portion length is between 2 mm and 30 mm, preferably between 5 mm and 15 mm. In accordance with an embodiment, said concave portion length is the height of the truncated cone delimiting the concave portion 109. In accordance with an embodiment, said concave portion length C is between 1/6 and ½ of the attachment distance D.

**.** In accordance with an embodiment, said intraluminal element 4 comprises a drug dispensing opening 4.

**.** Preferably, such an expandable balloon 105 is intended for the sclerosing treatment of varices.

**.** A manufacturing method of an expandable balloon 105 for medical-surgical applications having at least one concave portion 109 will be described below.

**.** A manufacturing method of an expandable balloon 105 for medical-surgical applications having at least one concave portion 109 comprises the steps of:
- including a mold cavity 113 having at least one concave portion 112;
- including a preform 120 or parison 120 for expandable balloons 105;
- fitting the parison 120 on the protrusion 114 of the die 110.

**.** In accordance with a preferred operating mode, the method comprises the step of overturning the parison 120 on the protrusion 114 of the die 110.

**.** In accordance with a preferred operating mode, the method comprises the step of inserting the parison 120 in the mold cavity 113.

**.** In accordance with a preferred operating mode, the method comprises the step of expanding the parison 120 in the concave portion 112 of the mold cavity 113.

**.** In accordance with a preferred operating mode, the method comprises the step of abutting the counter-die 131 against the die 110. If necessary, this allows closing the mold and firmly interposing a portion of the parison between the die 110 and the counter-die 131.

**.** In accordance with a possible operating mode, the step of providing a mold cavity 113 is performed by including a mold 100 according to any one of the embodiments described above. In accordance with a possible operating mode, the step of providing a mold cavity 113 is performed by including a die 110 according to any one of the embodiments described above.

**.** The parison 120 comprises an internal parison surface 125 delimiting an internal parison cavity 121 and an external parison surface 124 opposite said internal parison surface 125 facing the internal cavity 121.

**.** In accordance with an embodiment, the parison 120 extends longitudinally between a first parison end and a second parison end which define, respectively, an inlet opening and an outlet opening, preferably circular, with respect to the internal cavity 121. In accordance with an embodiment, said parison 120 has a substantially cylindrical shape.

**.** The step of fitting the parison 120 on the protrusion includes at least partially making a first parison surface portion 200 adhere to said protrusion. In accordance with an embodiment, said internal parison surface 125 or said external parison surface 124 comprises said first parison surface portion 200. In accordance with an embodiment, said first parison surface portion 200 is near said inlet opening or said outlet opening.

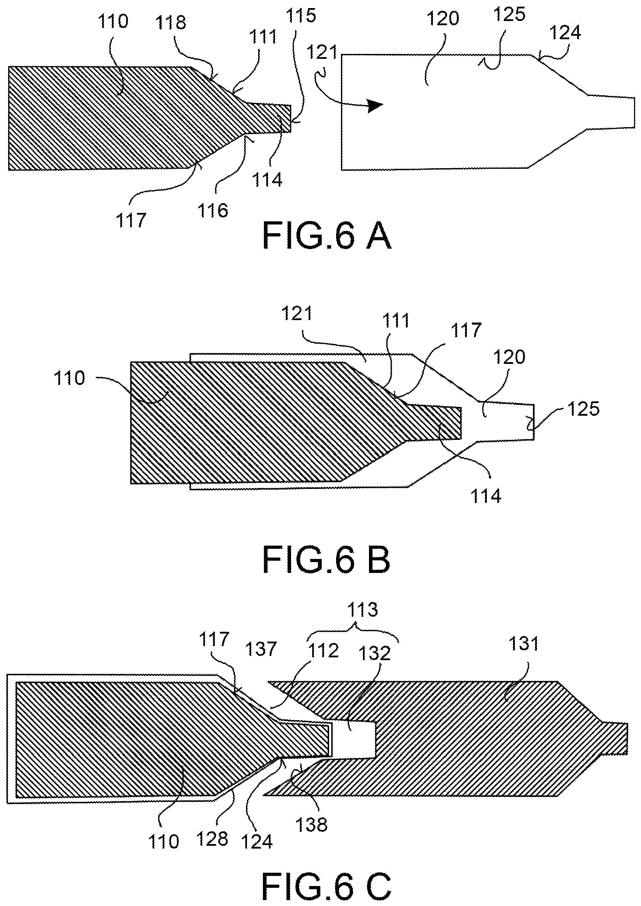

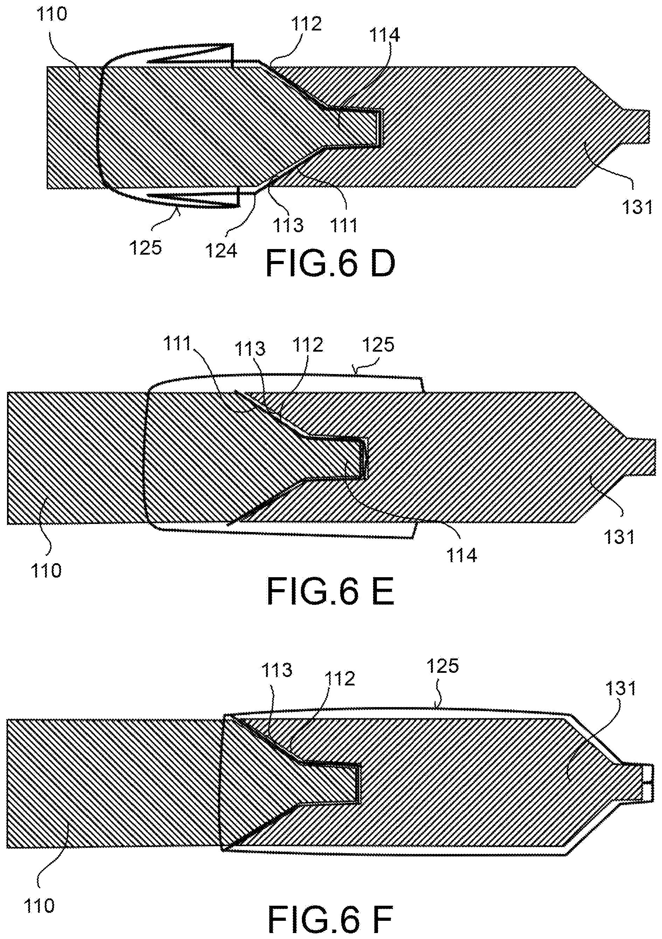

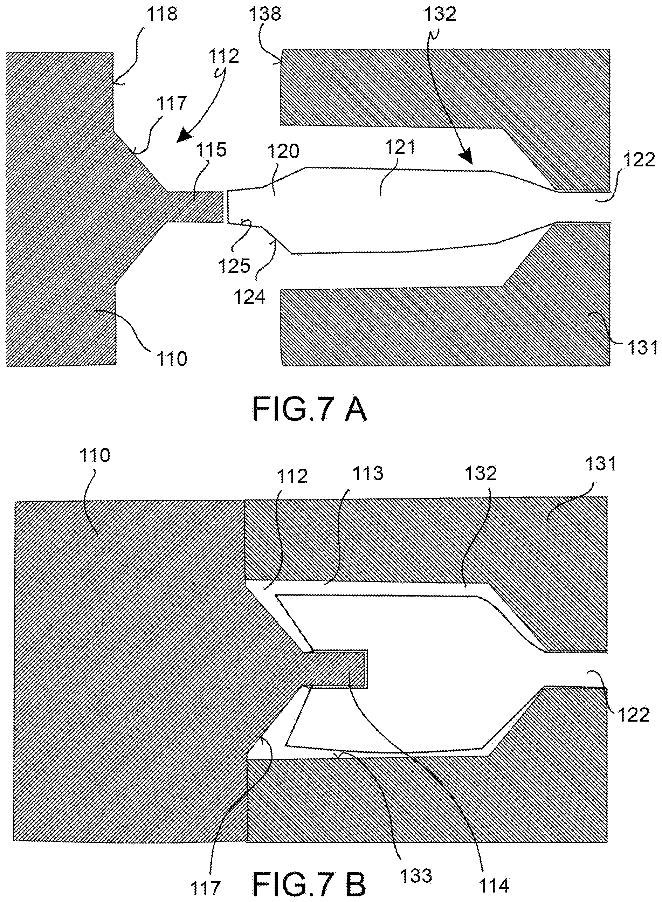

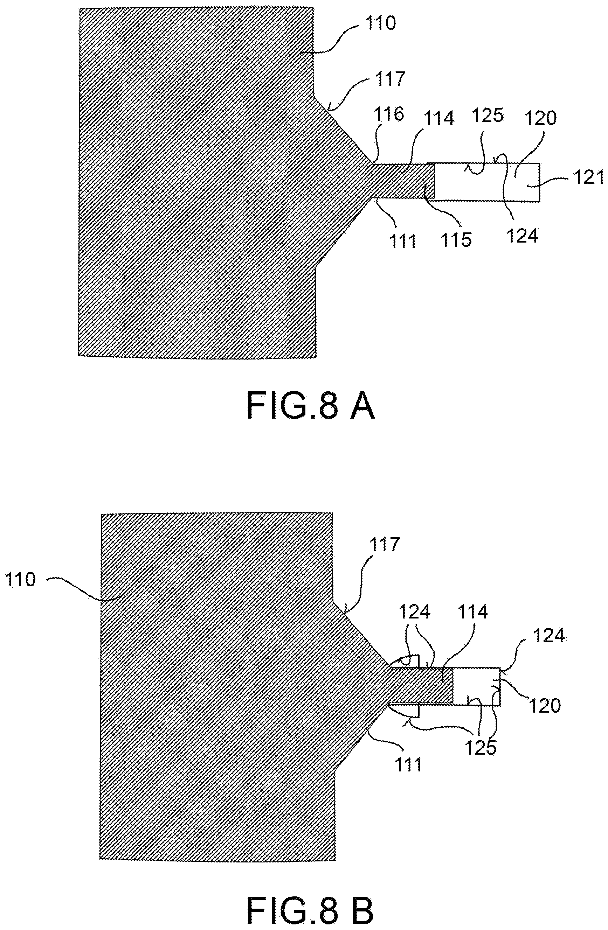

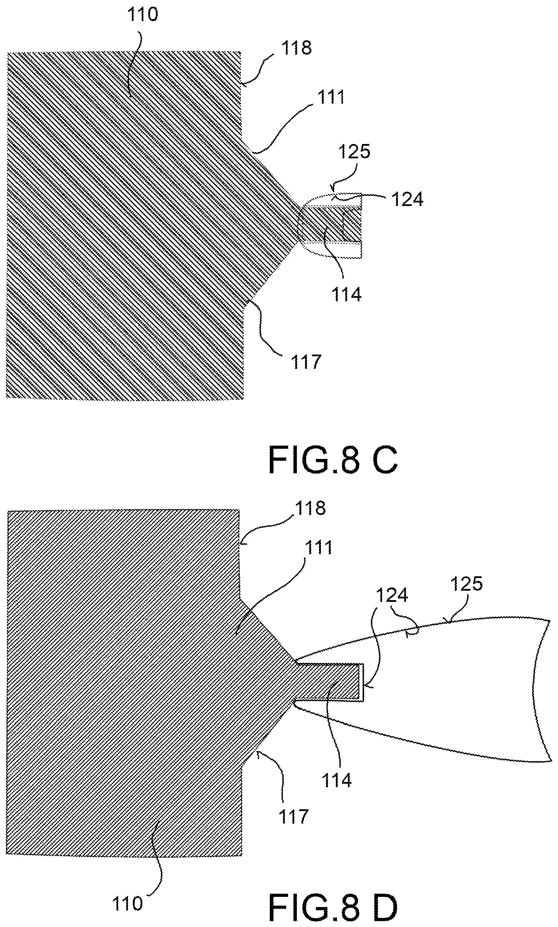

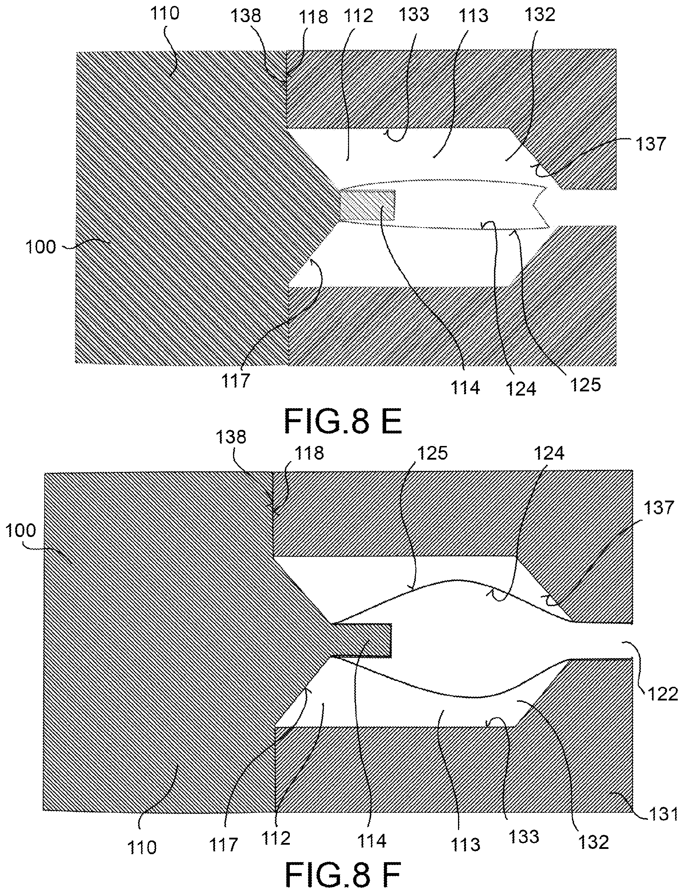

**.** In accordance with an operating mode, the method comprises the step of fitting the parison 120 on the protrusion 114. In accordance with an operating mode, the fitting step includes approaching the protrusion 114 with the external parison surface 124 of the parison 120, as shown for example in **figure 7-A.** In accordance with an operating mode, the fitting step includes approaching the protrusion 114 with the internal parison surface 125 of the parison 120, as shown for example in **figures 6-A**and **6-B** as well as in **figure 8-A**

**.** In accordance with an operating mode, the method comprises the step of overturning the parison 120 on the protrusion 114 of the die 110 of the mold 100, as shown for example in **figures 8-B**and **8-C.** By virtue of the overturning step, the same surface 124 or 125 of the parison 120 approaching the protrusion 114 of the die 110 is allowed to face the mold cavity 112 of the mold 100.

**.** In accordance with an operating method shown for example in **figures 6-A to 6-F**the parison 120 approaches the die 110, then the parison 120 receives the die 110 in the cavity 121 thereof so that the parison 120 is fitted on the first surface 111 of the die 110. Thereby, the internal parison surface 125 of the parison 120 faces the first surface 111 of the die 110. Subsequently, the counter-die 131 approaches the external parison surface 124 of the parison 120 fitted on the die 110. The counter-die 131 is thus abutted against the die 110. Preferably, the second tapered surface 137 of the counter-die 131 is abutted against the first tapered surface 117 of the die 110, so that a portion of the parison 120 is interposed therebetween and substantially locked. Subsequently, the parison 120 is overturned, bringing a free edge thereof on the counter-die 131. Thereby, the external parison surface 124 of the parison 120 faces the counter-die 131. Thereby, due to the effect of overturning the parison 120, it is possible to make an expandable balloon 105 having a concave portion 109.

**.** In accordance with an operating mode shown for example in **figures 7-A and 7-B****,** the parison 120 approaches the protrusion 114 of the die 110 with a closed margin thereof, i.e., unable to allow access to the cavity 121 and the parison 120 is shaped to the protrusion 114. The counter-die 131 is thus abutted against the die 110. The parison 120 is subsequently expanded by inflating through the channel 122. The shape of the concave portion 113 of the mold cavity 112 allows making an expandable balloon 105 having a concave portion 109.

**.** In accordance with an operating mode shown for example in **figures 8-A to 8-D**the parison 120 approaches the protrusion 114 of the die 110, then the parison 120 receives the protrusion 114 in the cavity 121 thereof, so that the parison 120 is fitted on the first surface 111 of the die 110. Thereby, the internal parison surface 125 of the parison 120 faces the first surface 111 of the die 110. Subsequently, the parison 120 is overturned. Subsequently, the parison 120 is overturned, bringing a free edge thereof on the protrusion 114, facing the internal parison surface 125 of the parison 120 to the mold cavity 112. The counter-die 131 is thus abutted against the die 110. The parison 120 is subsequently expanded by inflating through the channel 122. The shape of the concave portion 113 of the mold cavity 112 allows making an expandable balloon 105 having a concave portion 109.

**.** The method preferably comprises the step of heating the parison 120 as well.

**.** In accordance with an operating mode, the method includes the step of cooling the parison 120 to obtain the expandable balloon 105.

**.** In accordance with an operating mode, the method includes overturning the parison 120 on the protrusion 114 so that said internal parison surface 125 has at least one portion of said first parison surface portion 200 fitted on said protrusion 114 and so that the remaining part of said internal surface 125 faces said mold cavity 113.

**.** In accordance with an operating mode, the method includes circumferentially fastening the first parison end 120 to an intraluminal element 103 facing the entire said internal parison surface 125 to the intraluminal element 103. In accordance with an operating mode, the method includes overturning the parison 121 on the intraluminal element 103 so as to delimit the parison cavity 121 between the external parison surface 124 and the intraluminal element 103, fastening the second parison end from the opposite part to the first parison end. In accordance with an operating mode, the step of overturning the parison 120 includes leaving said first parison surface portion 200 of said internal parison surface 125 facing said intraluminal element 103, in which said fitting step of the parison 120 on the protrusion 114 includes at least partially fitting said first parison surface portion 200 on said protrusion 114,

**.** In accordance with an operating mode, the step of heating the parison 120 includes heating at least the first parison surface portion 200 at least simultaneously with the step of expanding the parison 120 in the mold cavity 113, by adhering said first parison surface portion 200 both on said protrusion 114 and on a first mold tapered surface 117 made integrally with said protrusion 114 so as to form the concave portion 109 of said expandable balloon 105. In accordance with an operating mode, said method includes heating and expanding the parison 200, by adhering a second parison surface portion 201 to an acute angle of the mold cavity 113 formed by the tapered surface 117 and the counter-die 131. In accordance with an operating mode, said step of cooling the parison 120 includes cooling said parison 120 while keeping the parison 120 expanded in the mold cavity. In accordance with an operating mode, said step of cooling the parison 120 includes cooling the second parison surface portion 201 so as to form a sharp contact portion 114 of said expandable balloon 105. In accordance with an operating mode, the first parison surface portion 200 comprises the second parison surface portion 201. In accordance with an operating mode, before said step of inserting the parison in the mold cavity, the first parison surface portion 200 is formed by a parison flap facing the intraluminal element 103.

**.** In accordance with an operating mode, said method includes the step of stretching the parison (120), for example moving said die 110 away from said counter-die 131.

**.** In accordance with an operating mode, the step of expanding the parison includes inflating the parison with a fluid passing through the intraluminal element 103.

**.** In accordance with an operating mode, said parison 120 is an elastomer or an extensible polymer. In accordance with an operating mode, said parison 120.

**.** In accordance with an operating mode, said step of heating the parison 120 includes heating the parison 120 in a temperature range between a glass transition temperature of the extensible polymer of the parison and a softening temperature or a melting temperature of the extensible polymer of the parison.

**.** In accordance with an operating mode, the step of cooling the parison 120 includes cooling the parison 120 to an ambient temperature between 15° and 25°.

**.** In accordance with an operating mode, said extensible polymer is a thermoplastic elastomer.

**.** In accordance with an operating mode, said extensible polymer comprises individually or in a mixture at least one of polyethylene, polyethylene terephthalate, polytetrafluoroethylene, polyamides, polyvinyl chloride, latex, silicones, polyurethane copolymers, polyamide copolymers, copolymers of polyamide and polyethers.

**.** In accordance with an operating mode, said parison 120 has a multilayer parison body, comprising a first elastomeric layer and a second thermoplastic layer. In accordance with an operating mode, said parison 120 comprises a non-compliant layer.

**.** In accordance with an operating mode, said parison 120 is made of a material adapted to make a compliant or semi-compliant expandable balloon.

**.** In accordance with an operating mode, the method comprises the step of making two convex balloons, and associating them with each other in a respective edge 126, 126' to form a single balloon having a concave portion 109, avoiding providing a mold having a concave portion 112. Preferably, the method comprises the step of providing two parisons 120, 120', each parison 120 and 120' being arranged in a respective pre-mold 123 and 123', and the step of expanding by inflating each parison 120 and 120' in the respective pre-mold 123 and 123', making two convex expandable balloons. Preferably, such pre-molds 123 and 123' are both convex, i.e., they are intended to form a convex balloon.

**.** The association between the edges 126 and 126' of the convex balloons to form a balloon having a concave portion 109 preferably occurs by laser welding.

**.** In accordance with an embodiment, such an association is performed by gluing. In accordance with an embodiment, such an association determines the formation of a concave portion 109 formed by one of the expandable balloons associated with each other in the respective edge 126, 126'.

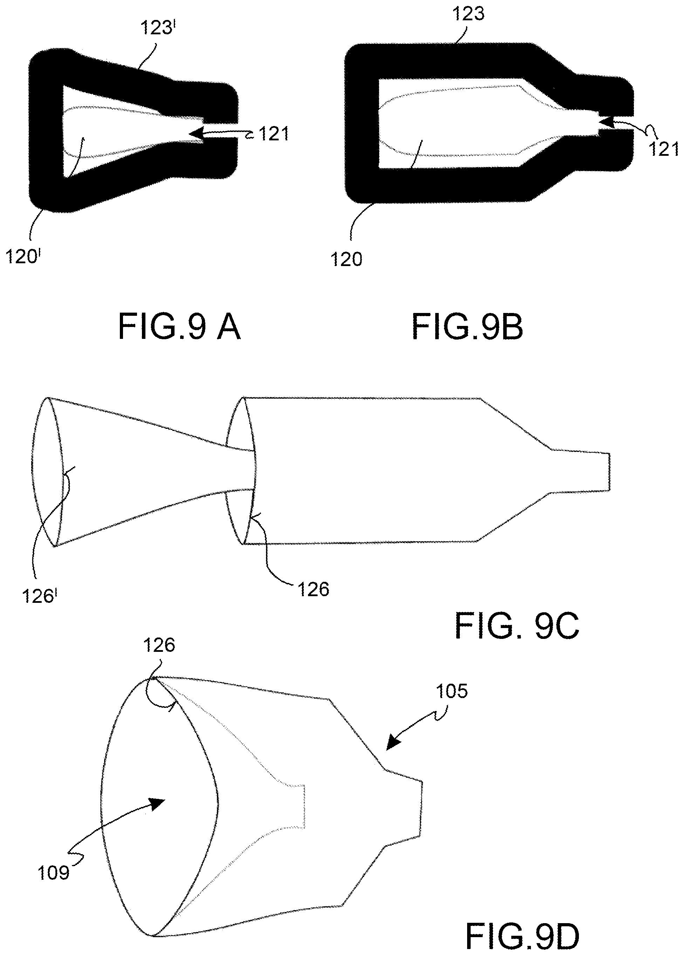

**.** Therefore, in accordance with a general embodiment, as shown for example in **figures 9-A to 9-D**a manufacturing method of an expandable balloon 105 for medical-surgical applications having at least one concave portion 109 comprises the steps of:
- making two substantially convex preforms;
- associating respective edges 126 and 126' of the convex preforms to each other, forming an expandable balloon 105 having a concave portion 109. Preferably, the concave portion 109 of the expandable balloon 105 is formed by one of the two convex preforms associated with each other. The preforms can for example be made as sheets of elastic material for expandable balloons.

**.** The preforms can in turn be expandable balloons.

**.** In accordance with an embodiment, the manufacturing method of an expandable balloon 105 for medical-surgical applications having at least one concave portion 109 comprises the steps of:
- making two convex balloons;
- associating respective edges 126 and 126' of the two convex balloons to each other, forming an expandable balloon 105 having a concave portion 109. Preferably, the concave portion 109 of the expandable balloon 105 is formed by one of the two associated convex balloons.

**.**

**.** A method for collecting material 107 from a lumen 102 of a patient's body will be described below.

**.** A method for collecting material 107 from the lumen 102 of a patient's body, such as from the lumen 102 of a blood vessel, comprises the steps of:
- including an expandable balloon 105 having a concave portion 109 at least when the expandable balloon 105 is in an expanded configuration;
- inserting said expandable balloon 105 inside the lumen 102 of the patient's body, for example inside a blood vessel 102;
- expanding the expandable balloon 105 so that it occupies a certain area of a transverse section of the lumen 102;
- advancing the expandable balloon 105 having the concave portion 109 in the lumen 102, facing the concave portion 109 toward the advancement direction X.

**.** The method preferably comprises the further step of extracting the expandable balloon 105 from the lumen 102 of the patient's body. This step is preferably performed with the concave portion 109 facing proximally and filled with collected material 107.

**.** The advancing step is preferably performed by keeping the concave portion 109 facing proximally.

**.** In accordance with a possible operating mode, the collection method is performed inside the lumen 102 of a blood vessel to collect material 107 which is formed as an effect of a pharmacological and/or abrasive treatment of the internal wall 106 of the blood vessel 102.

**.** For example, first a pharmacological treatment of the internal wall 106 of the blood vessel 102 is performed, subsequently or simultaneously an abrasive action is performed, for example mechanical, of the internal wall 106 of the blood vessel 102, and subsequently the collection method is performed.

**.** By virtue of the features described above included separately or jointly with each other in particular embodiments, it is possible to obtain a mold, as well as an expandable balloon, as well as a method which at the same time satisfy the above described requirements, contrasting each other, and the aforementioned desired advantages, and in particular:
- it allows making an expandable balloon provided with a concave portion;
- the concave portion has concavity facing a proximal or distal direction;

**.** In order to meet contingent and specific needs, those skilled in the art may make several changes and adaptations to the above-described embodiments, without however departing from the scope of the following claims.

**LIST OF REFERENCE NUMERALS**

| | |
|---|---|
| **4** | Drug dispensing opening |
| **6** | Blood vessel stretch wall |
| **9** | Balloon collecting portion |
| **14** | balloon contact portion |
| **17** | First balloon surface |
| **18** | Inflation fluid |
| **21** | Balloon reinforcing element or increased balloon section |
| **25** | First fastening element |
| **25'** | Second fastening element |
| **30** | Inflation opening |
| **100** | Mold |
| **101** | Vascular catheter |
| **102** | Lumen |
| **103** | Intraluminal shaft or element |
| **105** | Expandable balloon |
| **106** | Internal wall |
| **107** | Material |
| **109** | Concave balloon portion |
| **110** | Die |
| **111** | First die wall |
| **112** | Concave portion of the mold cavity |
| **113** | Mold cavity |
| **114** | Protrusion |
| **115** | Free end |
| **116** | Attachment root |
| **117** | Die tapered surface, or first tapered surface |
| **118** | Die abutment portion |
| **120** | Parison, or preform |
| **121** | Internal parison cavity |
| **122a** | First channel |
| **122b** | Second channel |
| **123** | Pre-mold |
| **124** | External parison surface |
| **125** | Internal parison surface |
| **126** | Edge |
| **131** | Counter-die |
| **132** | Convex portion of the mold cavity |
| **133** | Counter-die side wall |
| **137** | Counter-die tapered surface, or second tapered surface |
| **138** | Counter-die counter-abutment portion |
| **200** | First parison surface portion |
| **201** | Second parison surface portion |
| **X** | Advancement direction |
| **RO** | Radial direction |

## Claims

1. A mold (100) for forming an expandable balloon (105) for medical-surgical applications adapted to be fitted on an intraluminal element (103) comprising a die (110) and at least one counter-die (131),
wherein said die (110) comprises a first wall (111) at least partially delimiting a concave portion (112) of a mold cavity (113) configured to form at least one concave portion (109) of the expandable balloon (105) adapted to face said intraluminal element (103);
said first wall (111) comprising:
- a protrusion (114) adapted to project cantilevered in said mold cavity (113) forming a protrusion free end (115) and a protrusion root (116) opposite said protrusion free end;
- a first mold tapered surface (117) tapering as it approaches said protrusion root (116),
wherein the protrusion (114) and the first tapered surface (117) are made in a single piece,
wherein said counter-die (131) at least partially delimits a convex portion (132) of said mold cavity (113),
**characterized in that** said protrusion (114) has a substantially cylindrical shape,
and **in that** said tapered surface (117) and said protrusion (114) are jointed forming an obtuse angle.

2. A mold (100) according to claim 1, wherein the tapered surface (117) is frustoconical,
and/or wherein said die (110) and/or said counter-die (131) comprise heating elements.

3. A mold (100) according to any one of the preceding claims, wherein said counter-die (131) comprises a second tapered surface (137) tapering in a direction concordant with that of the first tapered surface (117) of the die (110).

4. A mold (100) according to the preceding claim, wherein said second tapered surface (137) of the counter-die (131) is parallel to the first tapered surface (117) of the die (110); and/or wherein
- said first tapered surface (117) and said second tapered surface (137) face each other and both face the mold cavity (113), delimiting therebetween a substantially concave volume having concavity facing the first tapered surface (117)
- wherein said counter-die (131) comprises at least one side wall (133) connected to the second tapered surface (137).

5. A mold (100) according to the preceding claim, wherein said at least one side wall (133) is substantially cylindrical, and/or wherein
said at least one side wall (133) at least partially delimits a portion of said mold cavity (113) between said first tapered surface (117) and said second tapered surface (137).

6. A mold (100) according to any one of the preceding claims, wherein said counter-die (131) forms an acute angle with the tapered surface (117), and/or wherein said counter-die (131) comprises a first channel wall delimiting the inlet of a first blowing channel (122b), wherein said first channel wall is connected to said second tapered surface (137) and/or wherein said die (110) comprises a second channel wall delimiting the inlet of a second inflation channel (122a), wherein said second channel of inflation channel (122a) is made inside said protrusion (114).

7. A manufacturing method of an expandable balloon (105) having at least one concave portion (109) comprises the steps of:
- providing a mold cavity (113) in a mold (100) according to any one of claims 1 to 6 having at least one concave portion (112) delimited by a first wall (111) having a protrusion (114) projecting into the mold cavity (113);
- providing a preform (120) or parison (120) for expandable balloons (105), wherein the parison (120) comprises an internal parison surface (125) delimiting an internal parison cavity (121) and an external parison surface (124) opposite said first surface (125) facing the internal cavity (121);
- inserting the parison (120) in the mold cavity (113);
- fitting the parison (120) on the protrusion (114), by at least partially adhering a first parison surface portion (200) of said internal surface (125) or of said external surface (124) at least on said protrusion (114).

8. A method according to claim 7, comprising the following further step:
- overturning the parison (120) on the protrusion (114) of the die (110) so that said internal surface (125) has at least one portion of said first parison surface (200) fitted on said protrusion (114) and the remaining part of said internal surface (125) facing said mold cavity (113).

9. A method according to claim 7, comprising the following further step:
- fastening a first end of the parison (120) to an intraluminal element (3), all of said internal parison surface (125) facing the intraluminal element (3), overturning the parison (121) on the intraluminal element (3) fastening a second parison end on the part opposite the first parison end so as to delimit the parison cavity (121) between the external parison surface (124) and the intraluminal element (3), leaving said first parison surface portion (200) of said internal parison surface (125) facing said intraluminal element (3), wherein said step of fitting the parison (120) on the protrusion (114) includes at least partially fitting said first parison surface portion (200) on said protrusion (114).

10. A method according to any one of the preceding claims 7 to 9, wherein the method comprises the further steps of
- expanding the parison (120) in the concave portion (112) of the mold cavity (113); and/or
- heating the parison (120), and/or
- cooling the parison (120) to obtain the expandable balloon (105).

11. A method according to the preceding claim, wherein said step of heating the parison (120) includes heating at least said first parison surface portion (200) at least simultaneously with the step of expanding the parison (120) in the mold cavity (113), by adhering said first parison surface portion (200) on said protrusion (114) and on a first mold tapered surface (117) made integrally with said protrusion (114) so as to form the concave portion (109) of said expandable balloon (105),
and wherein said step of cooling the parison (120) includes cooling said parison (120) while keeping the parison (120) expanded in the mold cavity,
and/or wherein said step of heating the parison (120) includes heating the parison (120) in a range of temperatures between a glass transition temperature and a softening temperature or a melting temperature;
and/or wherein the step of cooling the parison (120) includes cooling the parison (120) to room temperature.

12. A method according to any one of the preceding claims 7 to 11, wherein said parison (120) is an extensible polymer.

13. A method according to the preceding claim, wherein said extensible polymer is a thermoplastic elastomer,
and/or wherein said extensible polymer comprises, individually or in a mixture, at least one of polyethylene, polyethylene terephthalate, polytetrafluoroethylene, polyamides, polyvinyl chloride, latex, silicones, polyurethane copolymers or polyamides, copolymers of polyamides and polyethers.

14. A method according to any one of the preceding claims 7 to 13, wherein said parison (120) has a multilayer parison body, comprising a first elastomeric layer and a second thermoplastic layer.

## Patentansprüche

1. Form (100) zur Herstellung eines expandierbaren Ballons (105) für medizinische-chirurgische Anwendungen, die eingerichtet ist, an einem intraluminalen Element (103) angebracht zu werden, umfassend eine Matrize (110) und mindestens eine Gegenmatrize (131),
wobei die Matrize (110) eine erste Wand (111) umfasst, die mindestens teilweise einen konkaven Abschnitt (112) einer Formkavität (113) begrenzt, die dazu konfiguriert ist, mindestens einen konkaven Abschnitt (109) des expandierbaren Ballons (105) zu bilden, der eingerichtet ist, dem intraluminalen Element (103) zugewandt zu sein;
die erste Wand (111) umfassend:
- einen Vorsprung (114), der eingerichtet ist, in die Formkavität (113) auskragend vorzustehen, indem er ein freies Ende (115) des Vorsprungs und eine Basis (116) des Vorsprungs ausbildet, die dem freien Ende gegenüberliegt,
- eine erste verjüngte Formfläche (117), die sich verjüngt wenn sie sich der Basis (116) des Vorsprungs annähert,
wobei der Vorsprung (114) und die erste verjüngte Fläche (117) einstückig ausgebildet sind,
wobei die Gegenmatrize (131) mindestens teilweise einen konvexen Abschnitt (132) der Formkavität (113) begrenzt,
**dadurch gekennzeichnet, dass**
der Vorsprung (114) eine im Wesentlichen zylindrische Gestalt aufweist,
und dass
die verjüngte Fläche (117) und der Vorsprung (114) miteinander verbunden sind und einen stumpfen Winkel bilden.

2. Form (100) nach Anspruch 1, wobei die verjüngte Fläche (117) kegelstumpfförmig ausgebildet ist und/oder wobei die Matrize (110) und/oder die Gegenmatrize (131) Heizelemente umfassen.

3. Form (100) nach einem der vorhergehenden Ansprüche, wobei die Gegenmatrize (131) eine zweite verjüngte Fläche (137) umfasst, die sich in eine Richtung verjüngt, die mit der Richtung der ersten verjüngten Fläche (117) der Matrize (110) übereinstimmt.

4. Form (100) nach dem vorhergehenden Anspruch, wobei sich die zweite verjüngte Fläche (137) der Gegenmatrize (131) parallel zu der ersten verjüngten Fläche (117) der Matrize (110) erstreckt; und/oder wobei
- die erste verjüngte Fläche (117) und die zweite verjüngte Fläche (137) einander gegenüberliegen und beide der Formkavität (113) zugewandt sind, wobei sie dazwischen ein im Wesentlichen konkaves Volumen begrenzen, das eine Konkavität aufweist, die der ersten verjüngten Fläche (117) zugewandt ist,
- wobei die Gegenmatrize (131) mindestens eine Seitenwand (133) umfasst, die mit der zweiten verjüngten Fläche (137) verbunden ist.

5. Form (100) nach dem vorhergehenden Anspruch, wobei die mindestens eine Seitenwand (133) im Wesentlichen zylindrisch ist, und/oder wobei
die mindestens eine Seitenwand (133) mindestens teilweise einen Abschnitt der Formkavität (113) zwischen der ersten verjüngten Fläche (117) und der zweiten verjüngten Fläche (137) begrenzt.

6. Form (100) nach einem der vorhergehenden Ansprüche, wobei die Gegenmatrize (131) einen spitzen Winkel mit der verjüngten Fläche (117) bildet, und/oder wobei die Gegenmatrize (131) eine erste Kanalwand umfasst, die den Einlass eines ersten Blaskanals (122b) begrenzt, wobei die erste Kanalwand mit der zweiten verjüngten Fläche (137) verbunden ist und/oder wobei die Matrize (110) eine zweite Kanalwand umfasst, die den Einlass eines zweiten Blaskanals (122a) begrenzt, wobei der zweite Blaskanal (122a) innerhalb des Vorsprungs (114) ausgebildet ist.

7. Herstellungsverfahren eines expandierbaren Ballons (105) aufweisend mindestens einen konkaven Abschnitt (109), umfassend die folgenden Schritte:
- Bereitstellen einer Formkavität (113) in einer Form (100) nach einem der Ansprüche 1 bis 6 aufweisend mindestens einen konkaven Abschnitt (112), der durch eine erste Wand (111) aufweisend einen Vorsprung (114), der in die Formkavität (113) vorsteht, begrenzt wird,
- Bereitstellen eines Vorformlings (120) oder eines Parisons (120) für expandierbare Ballons (105), wobei der Parison (120) eine innere Parison-Oberfläche (125) umfasst, die eine innere Parison-Kavität (121) begrenzt, und eine äußere Parison-Oberfläche (124), die der ersten Oberfläche (125) gegenüberliegt und der inneren Kavität (121) zugewandt ist,
- Einführen des Parisons (120) in die Formkavität (113);
- Anbringen des Parisons (120) an dem Vorsprung (114), durch mindestens Anhaften teilweise eines ersten Parison-Oberflächenabschnitts (200) der inneren Oberfläche (125) oder der äußeren Oberfläche (124) mindestens auf den Vorsprung (114).

8. Verfahren nach Anspruch 7, umfassend den weiteren folgenden Schritt:
- Umkippen des Parisons (120) über den Vorsprung (114) der Matrize (110), so dass die innere Oberfläche (125) mindestens einen Abschnitt der ersten Parison-Oberfläche (200) aufweist, der auf dem Vorsprung (114) angebracht ist und der verbleibende Teil der inneren Oberfläche (125) der Formkavität (113) zugewandt ist.

9. Verfahren nach Anspruch 7, umfassend den weiteren folgenden Schritt:
- Befestigen eines ersten Endes des Parisons (120) an einem intraluminalen Element (3), wobei die ganze innere Parison-Oberfläche (125) dem intraluminalen Element (3) zugewandt ist, Umkippen des Parisons (121) über das intraluminale Element (3), Befestigen eines zweiten Endes des Parison an dem Teil des Parisons, der dem ersten Ende gegenüberliegt, sodass die Parison-Kavität (121) zwischen der äußeren Parison-Oberfläche (124) und dem intraluminalen Element (3) begrenzt ist, Zulassen, dass der erste Parison-Oberflächenabschnitt (200) der inneren Parison-Oberfläche (125) dem intraluminalen Element (3) zugewandt ist, wobei der Schritt des Aufbringens des Parisons (120) auf den Vorsprung (114) das Aufbringen mindestens teilweise des ersten Parison-Oberflächenabschnitts (200) auf den Vorsprung (114) umfasst.

10. Verfahren nach einem der vorhergehenden Ansprüche 7 bis 9, wobei das Verfahren die weiteren folgenden Schritte umfasst:
- Expandieren des Parisons (120) in dem konkaven Abschnitt (112) der Formkavität (113); und/oder
- Erhitzen des Parisons (120), und/oder
- Abkühlen des Parisos (120) zum Erhalten des expandierbaren Ballons (105).

11. Verfahren nach dem vorhergehenden Anspruch, wobei der Schritt des Erhitzens des Parisons (120) das Erhitzen mindestens des ersten Parison-Oberflächenabschnitts (200) mindestens gleichzeitig mit dem Schritt des Erweiterns des Parisons (120) in der Formkavität (113) umfasst, durch Anhaften des ersten Parison-Oberflächenabschnitts (200) auf dem Vorsprung (114) und auf einer ersten verjüngten Formfläche (117), die einstückig mit dem Vorsprung (114) ausgebildet ist, sodass den konkaven Abschnitt (109) des expandierbaren Ballons (105) zu bilden,
und wobei der Schritt des Abkühlens des Parisons (120) das Abkühlen des Parisons (120), indem der Parison (120) in dem expandierten Zustand in der Formkavität gehalten wird,
und/oder wobei der Schritt des Erhitzens des Parisons (120) das Erhitzen des Parisons (120) in einem Temperaturbereich zwischen einer Glasübergangstemperatur und einer Erweichungstemperatur oder einer Schmelztemperatur umfasst,
und/oder wobei der Schritt des Abkühlens des Parisons (120) das Abkühlen des Parisons (120) auf Raumtemperatur umfasst.

12. Verfahren nach einem der vorhergehenden Ansprüche 7 bis 11, wobei der Parison (120) ein dehnbarer Polymer ist.

13. Verfahren nach dem vorhergehenden Anspruch, wobei das dehnbare Polymer ein thermoplastisches Elastomer ist,
und/oder wobei das dehnbare Polymer, einzeln oder in einer Mischung, mindestens eines der folgenden umfasst: Polyethylen, Polyethylenterephthalat, Polytetrafluorethylen, Polyamide, Polyvinylchlorid, Latex, Silikone, Copolymere aus Polyurethan oder Polyamide, Copolymere aus Polyamiden und Polyethern.

14. Verfahren nach einem der vorhergehenden Ansprüche 7 bis 13, wobei der Parison (120) einen mehrschichtigen Parison-Körper aufweist, umfassend eine erste Elastomerschicht und eine zweite thermoplastische Schicht.

## Revendications

1. Moule (100) pour former un ballonnet expansible (105) pour des applications médico-chirurgicales, apte à être monté sur un élément intraluminal (103), comprenant une matrice (110) et au moins une contre-matrice (131),
dans lequel ladite matrice (110) comprend une première paroi (111) délimitant au moins partiellement une portion concave (112) d'une cavité de moule (113) configurée pour former au moins une portion concave (109) du ballonnet expansible (105) apte à faire face audit élément intraluminal (103) ;
ladite première paroi (111) comprenant :
- une saillie (114) apte à s'étendre en porte-à-faux dans ladite cavité de moule (113) formant une extrémité libre de saillie (115) et une base de saillie (116) opposée à ladite extrémité libre ;
- une première surface conique de moule (117) s'amincissant à l'approche de ladite base de saillie (116),
dans lequel la saillie (114) et la première surface conique (117) sont réalisées en une seule pièce,
dans lequel ladite contre-matrice (131) délimite au moins partiellement une portion convexe (132) de ladite cavité de moule (113),
**caractérisé en ce que**
ladite saillie (114) a une forme sensiblement cylindrique, et **en ce que**
ladite surface conique (117) et ladite saillie (114) sont j ointes formant un angle obtus.

2. Moule (100) selon la revendication 1, dans lequel la surface conique (117) est tronconique, et/ou dans lequel ladite matrice (110) et/ou ladite contre-matrice (131) comprend des éléments chauffants.

3. Moule (100) selon l'une quelconque des revendications précédentes, dans lequel ladite contre-matrice (131) comprend une seconde surface conique (137) s'amincissant dans une direction concordante avec celle de la première surface conique (117) de la matrice (110).

4. Moule (100) selon la revendication précédente, dans lequel ladite seconde surface conique (137) de la contre-matrice (131) est parallèle à la première surface conique (117) de la matrice (110) ; et/ou dans lequel
- ladite première surface conique (117) et ladite seconde surface conique (137) se font face et sont toutes deux orientées vers la cavité de moule (113), délimitant entre elles un volume sensiblement concave ayant une concavité orientée vers la première surface conique (117),
- dans lequel ladite contre-matrice (131) comprend au moins une paroi latérale (133) reliée à la seconde surface conique (137).

5. Moule (100) selon la revendication précédente, dans lequel ladite au moins une paroi latérale (133) est sensiblement cylindrique, et/ou dans lequel
ladite au moins une paroi latérale (133) délimite au moins partiellement une portion de ladite cavité de moule (113) entre ladite première surface conique (117) et ladite seconde surface conique (137).

6. Moule (100) selon l'une quelconque des revendications précédentes, dans lequel ladite contre-matrice (131) forme un angle aigu avec la surface conique (117), et/ou dans lequel ladite contre-matrice (131) comprend une première paroi de canal délimitant l'entrée d'un premier canal de soufflage (122b), dans lequel ladite première paroi de canal est reliée à ladite seconde surface conique (137), et/ou dans lequel ladite matrice (110) comprend une seconde paroi de canal délimitant l'entrée d'un second canal de gonflage (122a), dans lequel ledit second canal de gonflage (122a) est réalisé à l'intérieur de ladite saillie (114).

7. Procédé de fabrication d'un ballonnet expansible (105) présentant au moins une portion concave (109), comprenant les étapes consistant à :
- prévoir une cavité de moule (113) dans un moule (100) selon l'une quelconque des revendications 1 à 6, ayant au moins une portion concave (112) délimitée par une première paroi (111) ayant une saillie (114) s'étendant dans la cavité de moule (113) ;
- prévoir une préforme (120) ou paraison (120) pour ballonnets expansibles (105), dans laquelle la paraison (120) comprend une surface interne de paraison (125) délimitant une cavité interne de paraison (121) et une surface externe de paraison (124) opposée à ladite première surface (125) faisant face à la cavité interne (121) ;
- introduire la paraison (120) dans la cavité de moule (113) ;
- monter la paraison (120) sur la saillie (114), en faisant adhérer au moins partiellement une première portion de surface de paraison (200) de ladite surface interne (125) ou de ladite surface externe (124) au moins sur ladite saillie (114).

8. Procédé selon la revendication 7, comprenant l'étape supplémentaire suivante :
- renverser la paraison (120) sur la saillie (114) de la matrice (110) de sorte que ladite surface interne (125) présente au moins une portion de ladite première surface de paraison (200) montée sur ladite saillie (114) et que la partie restante de ladite surface interne (125) soit orientée vers ladite cavité de moule (113).

9. Procédé selon la revendication 7, comprenant l'étape supplémentaire suivante :
- fixer une première extrémité de la paraison (120) à un élément intraluminal (3), l'ensemble de ladite surface interne de paraison (125) étant orientée vers l'élément intraluminal (3), renverser la paraison (121) sur l'élément intraluminal (3), fixer une seconde extrémité de la paraison sur la partie opposée à la première extrémité de la paraison de manière à délimiter la cavité de paraison (121) entre la surface externe de paraison (124) et l'élément intraluminal (3), en laissant ladite première portion de surface de paraison (200) de ladite surface interne de paraison (125) orientée vers ledit élément intraluminal (3), dans lequel ladite étape de montage de la paraison (120) sur la saillie (114) comprend le montage au moins partiel de ladite première portion de surface de paraison (200) sur ladite saillie (114).

10. Procédé selon l'une quelconque des revendications 7 à 9, dans lequel le procédé comprend les étapes supplémentaires consistant à :
- expandre la paraison (120) dans la portion concave (112) de la cavité de moule (113) ; et/ou
- chauffer la paraison (120), et/ou
- refroidir la paraison (120) pour obtenir le ballonnet expansible (105).

11. Procédé selon la revendication précédente, dans lequel ladite étape de chauffage de la paraison (120) comprend le chauffage d'au moins ladite première portion de surface de paraison (200), au moins simultanément avec l'étape d'expansion de la paraison (120) dans la cavité de moule (113), en faisant adhérer ladite première portion de surface de paraison (200) sur ladite saillie (114) et sur une première surface conique de moule (117) réalisée d'un seul tenant avec ladite saillie (114) de manière à former la portion concave (109) dudit ballonnet expansible (105),
et dans lequel ladite étape de refroidissement de la paraison (120) comprend le refroidissement de ladite paraison (120) tout en la maintenant expandue dans la cavité de moule,
et/ou dans lequel ladite étape de chauffage de la paraison (120) comprend le chauffage de ladite paraison (120) dans une plage de températures comprise entre une température de transition vitreuse et une température d'assouplissement ou de fusion ;
et/ou dans lequel l'étape de refroidissement de la paraison (120) comprend le refroidissement de ladite paraison (120) jusqu'à température ambiante.

12. Procédé selon l'une quelconque des revendications 7 à 11, dans lequel ladite paraison (120) est un polymère extensible.

13. Procédé selon la revendication précédente, dans lequel ledit polymère extensible est un élastomère thermoplastique,
et/ou dans lequel ledit polymère extensible comprend, individuellement ou en mélange, au moins un parmi polyéthylène, téréphtalate de polyéthylène, polytétrafluoroéthylène, polyamides, chlorure de polyvinyle, latex, silicones, copolymères de polyuréthane ou polyamides, copolymères de polyamides et polyéthers.

14. Procédé selon l'une quelconque des revendications 7 à 13, dans lequel ladite paraison (120) présente un corps de paraison multicouche, comprenant une première couche élastomère et une seconde couche thermoplastique.
